(19) Europäisches Patentamt
European Patent Office
Office européen des brevets

(11) Veröffentlichungsnummer: **0 379 894 A2**

# (12) EUROPÄISCHE PATENTANMELDUNG

(21) Anmeldenummer: **90100504.1**

(22) Anmeldetag: **11.01.90**

(51) Int. Cl.5: **C07D 207/452, C07D 231/16, C07D 265/12, //C07D413/04, A01N43/84**

(30) Priorität: **24.01.89 DE 3901901**

(43) Veröffentlichungstag der Anmeldung: **01.08.90 Patentblatt 90/31**

(84) Benannte Vertragsstaaten: **BE CH DE FR GB IT LI NL**

(71) Anmelder: **BAYER AG**

**D-5090 Leverkusen 1 Bayerwerk(DE)**

(72) Erfinder: **Lantzsch, Reinhard, Dr.**
**Am Buschhaeuschen 51**
**D-5600 Wuppertal 1(DE)**
Erfinder: **Jensen-Korte, Uta, Dr.**
**Geibelstrasse 9**
**D-4000 Düsseldorf 1(DE)**
Erfinder: **Schallner, Otto, Dr.**
**Noldeweg 22**
**D-4019 Monheim(DE)**
Erfinder: **Kysela, Ernst, Dr.**
**Virchowstrasse 14**
**D-5060 Bergisch-Gladbach 1(DE)**

## (54) Verfahren zur Herstellung von (5-Fluor-2-nitro-4-heterocyclyl-phenoxy)-essigsäure-Derivaten und neue Zwischenprodukte.

(57) Die Erfindung betrifft ein neues Verfahren zur Herstellung von (5-Fluor-2-nitro-4-heterocyclyl-phenoxy)-essigsäure-Derivaten der allgemeinen Formel (I)

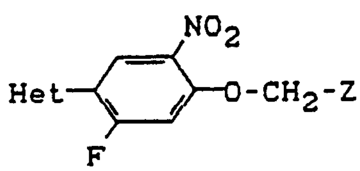

(I)

in welcher
Z für Cyano, Carboxy oder Alkoxycarbonyl steht und
Het für eine der nachstehenden heterocyclischen Gruppierungen steht

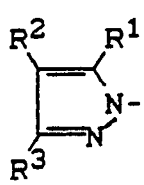 , 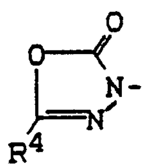 , 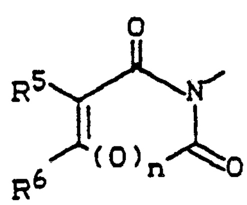

wobei die Substituenten die in der Beschreibung angegebene Bedeutung haben, dadurch gekennzeichnet, daß man 1-Heterocyclyl-2,4-difluor-5-nitrobenzol-Derivate der allgemeinen Formel (II)

EP 0 379 894 A2

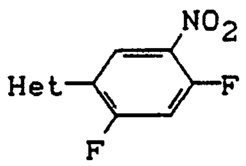

(II)

in welcher
Het die oben angegebene Bedeutung hat, mit Hydroxyessigsäure-Derivaten der Formel (III)
$HO-CH_2-Z$ (III)
oder deren Alkalimetallsalzen umsetzt.

## Verfahren zur Herstellung von (5-Fluor-2-nitro-4-heterocyclyl-phenoxy)-essigsäure-Derivaten und neue Zwischenprodukte

Die Erfindung betrifft ein neues Verfahren zur Herstellung von (5-Fluor-2-nitro-4-heterocyclyl-phenoxy)-essigsäure-Derivaten, welche als Zwischenprodukte für die Herstellung von Herbiziden verwendet werden können, sowie neue 1-Heterocyclyl-2,4-difluor-5-nitro-benzolDerivate als Zwischenprodukte hierfür und ein Verfahren zu deren Herstellung.

Es ist bekannt, daß man bestimmte (2-Nitro-4-heterocyclyl-phenylamino)-essigsäureester, wie z.B. 2-(4-Methoxycarbonyl-methylamino-3-nitro-phenyl)-4,5,6,7-tetrahydro-2H-isoindol-1,3-dion erhält, wenn man entsprechende 1-Fluor-2-nitro-4-heterocyclyl-benzol-Derivate, wie z.B. 2-(4-Fluor-3-nitro-phenyl)-4,5,6,7-tetrahydro-2H-isoindol-1,3-dion, mit Aminoessigsäureestern bzw. deren Hydrochloriden, wie z.B. Glycin-methylester-Hydrochlorid, in Gegenwart von Basen, wie z.B. Triethylamin, umsetzt (vgl. EP-A 177 032, Beispiel 2).

Es wurde nun gefunden, daß man (5-Fluor-2-nitro-4-heterocyclyl-phenoxy)-essigsäure-Derivate der allgemeinen Formel (I)

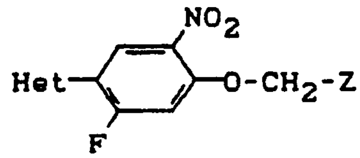

(I)

in welcher

Z für Cyano, Carboxy oder Alkoxycarbonyl steht und

Het für eine der nachstehenden heterocyclischen Gruppierungen steht

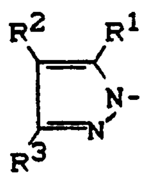
,
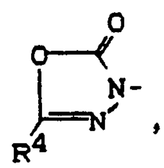
,
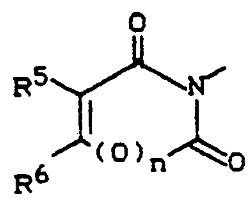

worin

$R^1$ für Wasserstoff, Halogen oder gegebenenfalls durch Halogen substituiertes Alkyl steht,

$R^2$ für Wasserstoff, Cyano, Nitro, Halogen oder gegebenenfalls durch Halogen substituiertes Alkyl steht,

$R^3$ für Wasserstoff oder gegebenenfalls durch Halogen substituiertes Alkyl steht oder zusammen mit $R^2$ für Alkandiyl steht,

$R^4$ für Wasserstoff oder für jeweils gegebenenfalls verzweigtes und gegebenenfalls durch Halogen substituiertes Alkyl, Cycloalkyl, Alkenyl oder Alkinyl steht,

$R^5$ für Wasserstoff, Halogen oder gegebenenfalls durch Halogen substituiertes Alkyl steht,

$R^6$ für Wasserstoff, Halogen oder gegebenenfalls durch Halogen substituiertes Alkyl steht, oder zusammen mit $R^5$ für Alkandiyl steht und

n für die Zahlen 0 oder 1 steht,

in guten Ausbeuten und in hoher Reinheit erhält, wenn man 1-Heterocyclyl-2,4-difluor-5-nitrobenzol-Derivate der allgemeinen Formel (II)

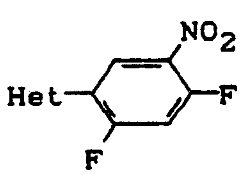

(II)

in welcher

Het die oben angegebene Bedeutung hat,
mit Hydroxyessigsäure-Derivaten der allgemeinen Formel (III)
$HO-CH_2-Z$ (III)
in welcher
Z die oben angegebene Bedeutung hat,
oder mit Alkalimetallsalzen von Verbindungen der Formel (III)
in Gegenwart eines Verdünnungsmittels und gegebenenfalls in Gegenwart eines Säureakzeptors bei Temperaturen zwischen -20 °C und + 100 °C umsetzt.

Es ist als überraschend anzusehen, daß beim erfindungsgemäßen Verfahren eine nucleophile Substitution praktisch nur in ortho-Position zur Nitrogruppe erfolgt, da bei Vorhandensein von Halogen in ortho- und in para-Position zur Nitrogruppe deren Substitution zu etwa gleichen Teilen zu erwarten war.

Verwendet man beispielsweise 1-(4-Chlor-pyrazol-1-yl)-2,4-difluor-5-nitro-benzol und Glycolsäuremethylester als Ausgangsstoffe, so kann der Reaktionsablauf beim erfindungsgemäßen Verfahren durch das folgende Formelschema wiedergegeben werden:

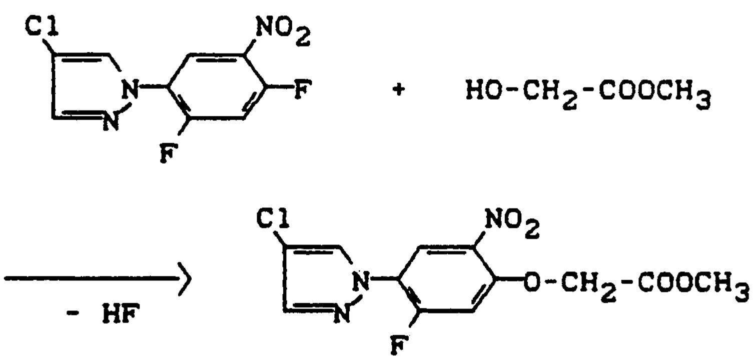


Nach dem erfindungsgemäßen Verfahren werden vorzugsweise Verbindungen der Formel (I) hergestellt, in welcher
Z für Cyano, Carboxy oder $C_1$-$C_4$-Alkoxycarbonyl steht und
Het für eine der nachstehenden heterocyclischen Gruppierungen steht

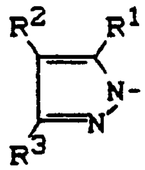 , 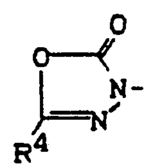 , 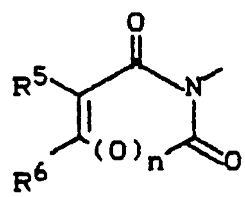

worin
$R^1$ für Wasserstoff, Fluor, Chlor, Brom oder für gegebenenfalls durch Fluor und/oder Chlor substituiertes $C_1$-$C_4$-Alkyl steht,
$R^2$ für Wasserstoff, Cyano, Nitro, Fluor, Chlor, Brom, Iod oder für gegebenenfalls durch Fluor und/oder Chlor substituiertes $C_1$-$C_4$-Alkyl steht,
$R^3$ für Wasserstoff oder für gegebenenfalls durch Fluor und/oder Chlor substituiertes $C_1$-$C_4$-Alkyl steht, oder zusammen mit $R^2$ für $C_3$- oder $C_4$-Alkandiyl (Trimethylen oder Tetramethylen) steht,
$R^4$ für Wasserstoff oder für jeweils gegebenenfalls verzweigtes und gegebenenfalls durch Fluor, Chlor und/oder Brom substituiertes $C_1$-$C_4$-Alkyl, $C_3$-$C_6$-Cycloalkyl, $C_2$-$C_4$-Alkenyl oder $C_2$-$C_4$-Alkinyl steht,
$R^5$ für Wasserstoff, Fluor, Chlor, Brom oder für gegebenenfalls durch Fluor und/oder Chlor substituiertes $C_1$-$C_4$-Alkyl steht,
$R^6$ für Wasserstoff, Fluor, Chlor, Brom oder für gegebenenfalls durch Fluor und/oder Chlor substituiertes $C_1$-$C_4$-Alkyl steht, oder zusammen mit $R^5$ für $C_3$- oder $C_4$-Alkandiyl (Trimethylen oder Tetramethylen) steht und
n für die Zahlen 0 oder 1 steht.

Insbesondere werden nach dem erfindungsgemäßen Verfahren Verbindungen der Formel (I) hergestellt, in welcher

Z für Methoxycarbonyl, Ethoxycarbonyl, n- oder iso-Propoxycarbonyl, n-, iso-, sec- oder tert. Butoxycarbonyl steht und
Het für eine der nachstehenden heterocyclischen Gruppierungen steht

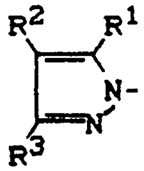 , 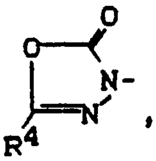 , 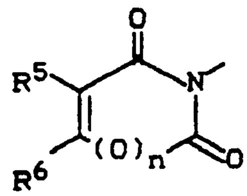

worin
$R^1$ für Wasserstoff, Chlor, Methyl, Ethyl oder Trifluormethyl steht,
$R^2$ für Wasserstoff, Chlor, Brom, Methyl oder Trifluormethyl steht,
$R^3$ für Wasserstoff, Methyl, Ethyl oder Trifluormethyl steht, oder zusammen mit $R^2$ für Trimethylen oder Tetramethylen steht,
$R^4$ für Wasserstoff oder für jeweils gegebenenfalls verzweigtes und gegebenenfalls durch Fluor und/oder Chlor substituiertes $C_1$-$C_4$-Alkyl oder $C_3$-$C_5$-Cycloalkyl steht, inbesondere für Wasserstoff oder jeweils durch Fluor und/oder Chlor substituiertes Methyl, Ethyl, n- oder iso-Propyl, Cyclopropyl oder Cyclopentyl steht,
$R^5$ für Wasserstoff, Chlor oder Methyl steht,
$R^6$ für Wasserstoff, Chlor oder Methyl steht, oder zusammen mit $R^5$ für Trimethylen oder Tetramethylen steht und
n für die Zahlen 0 oder 1 steht.

Die bei dem erfindungsgemäßen Verfahren zur Herstellung von Verbindungen der Formel (I) als Ausgangsstoffe zu verwendenden 1-Heterocyclyl-2,4-difluor-5-nitrobenzol-Derivate sind durch die Formel (II) allgemein definiert.

In Formel (II) hat Het vorzugsweise bzw. insbesondere diejenige Bedeutung, die bereits oben im Zusammenhang mit der Beschreibung der Verbindungen der Formel (I) vorzugsweise bzw. als insbesondere bevorzugt für Het angegeben wurde.

Als Beispiele für die Ausgangsstoffe der Formel (II) seien genannt: Pyrazol-1-yl-, (4-Chlor-pyrazol-1-yl)-, (4-Brom-pyrazol-1-yl)-, (4-Methyl-pyrazol-1-yl)-, (3,5-Dimethyl-pyrazol-1-yl)-, (3,4,5-Trimethyl-pyrazol-1-yl)-, (4-Chlor-3,5-dimethyl-pyrazol-1-yl)-, (4-Brom-3,5-dimethyl-pyrazol-1-yl)-, (3,4-Trimethylen-pyrazol-1-yl)-, (3,4-Tetramethylenpyrazol-1-yl)-, (5-Chlor-3,4-trimethylen-pyrazol-1-yl)-, (5-Methyl-3,4-trimethylen-pyrazol-1-yl)-, (5-Chlor-3,4-tetramethylen-pyrazol-1-yl)-, (5-Methyl-3,4-tetramethylen-pyrazol-1-yl)-, (5-Oxo-1,3,4-oxadiazol-4-yl)-, (2-Methyl-5-oxo-1,3,4-oxadiazol-4-yl)-, (2-Ethyl-5-oxo-1,3,4-oxadiazol-4-yl)-, (2-Propyl-5-oxo-1,3,4-oxadiazol-4-yl)-, (2-Isopropyl-5-oxo-1,3,4-oxadiazol-4-yl)-, (2-Butyl-5-oxo-1,3,4-oxadiazol-4-yl)-, (2-Isobutyl-5-oxo-1,3,4-oxadiazol-4-yl)-, (2-sec-Butyl-5-oxo-1,3,4-oxadiazol-4-yl)-, (2-tert-Butyl-5-oxo-1,3,4-oxadiazol-4-yl)-, (3,4-Dimethyl-2,5-dioxo-2,5-dihydro-pyrrol-1-yl)-, (3,4-Trimethylen-2,5-dioxo-2,5-dihydro-pyrrol-1-yl)-, (3,4-Tetramethylen2,5-dioxo-2,5-dihydro-pyrrol-1-yl)-, (5,6-Dimethyl-2,4-dioxo-3,4-dihydro-2H-1,3-oxazin-3-yl)-, (5,6-Trimethylen-2,4-dioxo-2,4-dihydro-2H-1,3-oxazin-3-yl)- und (5,6-Tetramethylen-2,4-dioxo-2,4-dihydro-2H-1,3-oxazin-3-yl)-2,4-difluor-5-nitro-benzol.

Die 1-Heterocyclyl-2,4-difluor-5-nitro-benzol-Derivate der Formel (II) sind noch nicht aus der Literatur bekannt und Gegenstand der vorliegenden Erfindung.

Man erhält die neuen Verbindungen der Formel (II), wenn man 1-Heterocyclyl-2,4-difluor-benzol-Derivate der allgemeinen Formel (IV)

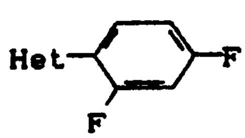 (IV)

in welcher
Het die oben angegebene Bedeutung hat,
mit Nitrierungsmitteln, wie z.B. Salpetersäure, gegebenenfalls in Gegenwart von Schwefelsäure, bei Temperaturen zwischen - 20 °C und + 50 °C umsetzt und nach üblichen Methoden aufarbeitet.

Für den Fall, daß Het für die Gruppierung

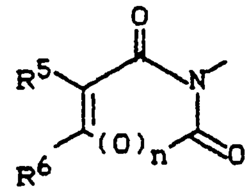

steht, worin
$R^5$ und $R^6$ die oben angegebene Bedeutung haben und n für die Zahl 0 steht,
erhält man die Verbindungen der Formel (II) auch, wenn man cyclische Anhydride der Formel (V)

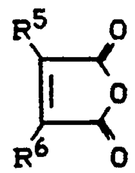

(V)

in welcher
$R^5$ und $R^6$ die oben angegebene Bedeutung haben,
mit 2,4-Difluor-5-nitro-anilin, gegebenenfalls in Gegenwart eines Verdünnungsmittels, wie z.B. Essigsäure, bei Temperaturen zwischen 20 °C und 150 °C umsetzt und nach üblichen Methoden aufarbeitet.

Die als Zwischenprodukte zu verwendenden 1-Heterocyclyl-2,4-difluor-benzol-Derivate der Formel (IV) sind bekannt und/oder können nach an sich bekannten Verfahren hergestellt werden (vgl. DE-OS 26 46 628, DE-OS 27 01 467, US-P 40 32 326, US-P 40 84 055, US-P 41 08 628).

Für den Fall, daß Het für die Gruppierung

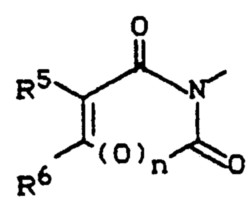

steht, worin
$R^5$ und $R^6$ die oben angegebene Bedeutung hat und n für die Zahl 0 steht,
erhält man die Verbindungen der Formel (IV), wenn man cyclische Anhydride der Formel (V)

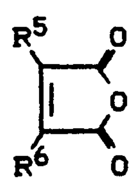

(V)

in welcher
$R^5$ und $R^6$ die oben angegebene Bedeutung haben,
mit 2,4-Difluor-anilin, gegebenenfalls in Gegenwart eines Verdünnungsmittels, wie z.B. Essigsäure, bei Temperaturen zwischen 20 °C und 150 °C umsetzt und nach üblichen Methoden aufarbeitet.

Für den Fall, daß Het für die Gruppierung

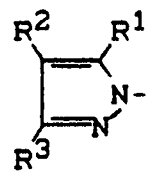

steht, worin

$R^1$, $R^2$ und $R^3$ die oben angegebene Bedeutung haben,

erhält man die Verbindungen der Formel (IV), wenn man 1,3-Dicarbonylverbindungen der Formel (VI)

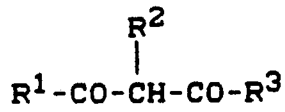

(VI)

in welcher

$R^1$ $R^2$ und $R^3$ die oben angegebene Bedeutung haben,

mit 2,4-Difluor-phenylhydrazin, gegebenenfalls in Gegenwart eines Verdünnungsmittel, wie z.B. Ethanol, bei Temperaturen zwischen 20 °C und 100 °C umsetzt und nach üblichen Methoden aufarbeitet.

Die weiter als Zwischenprodukte zu verwendenden cyclischen Anhydride der Formel (V) sind bekannte organische Synthesechemikalien.

Das gegebenenfalls als Zwischenprodukt einzusetzende 2,4-Difluor-5-nitro-anilin ist noch nicht aus der Literatur bekannt. Man erhält diese Verbindung, wenn man 2,4-Difluor-anilin mit einem Nitrierungsmittel, wie Salpetersäure, vorzugsweise in Gegenwart von Schwefelsäure, bei Temperaturen zwischen -20 °C und +20 °C umsetzt und nach üblichen Methoden aufarbeitet.

2,4-Difluor-anilin und 2,4-Difluorphenylhydrazin sind bekannte Synthesechemikalien.

Die bei dem erfindungsgemäßen Verfahren zur Herstellung von Verbindungen der Formel (I) weiter als Ausgangsstof fe zu verwendenden Hydroxyessigsäure-Derivate sind durch die Formel (III) allgemein definiert.

In Formel (III) hat Z vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für Z angegeben wurde.

Als Beispiele für die Ausgangsstoffe der Formel (III) seien genannt: Hydroxyessigsäure-methylester, -ethylester, -propylester, -isopropylester, -butylester, -isobutylester, -sec-butylester und -tert-butylester.

Die Hydroxyessigsäure-Derivate der Formel (III) sind bekannt und/oder können nach an sich bekannten Verfahren hergestellt werden.

Das erfindungsgemäße Verfahren zur Herstellung der Verbindungen der Formel (I) wird unter Verwendung von Verdünnungsmitteln durchgeführt. Als Verdünnungsmittel kommen praktisch alle inerten organischen Lösungsmittel infrage. Hierzu gehören insbesondere Ether, wie Diethylether, Dipropylether, Diisopropylether, Dibutylether, Diisobutylether, Methyl-tert-butylether, Methyl-tert.-amylether, 1,2-Dimethoxyethan, Diethylenglycoldimethylether, Tetrahydrofuran und Dioxan, gegebenenfalls halogenierte Kohlenwasserstoffe, wie Toluol, Xylol, Chlorbenzol, Cyclohexan und Methylcyclohexan, Nitrile, wie Acetonitril oder Propionitril, oder weitere aprotische polare Solventien, wie Dimethylformamid, Dimethylacetamid, N-Methylpyrrolidon, Dimethylsulfoxid und Sulfolan. Besonders bevorzugt verwendet man Tetrahydrofuran und Dioxan.

Das erfindungsgemäße Verfahren wird vorzugsweise in Gegenwart von Säureakzeptoren durchgeführt. Als solche kommen insbesondere Alkalimetallhydride, -hydroxide, -carbonate und -alkoholate infrage, wie z.B. Lithium-, Natrium- und Kalium-hydrid, Lithium-, Natrium- und Kalium-hydroxid, Lithium-, Natrium- und Kalium-carbonat sowie Lithium-, Natrium- und Kalium-methylat, -ethylat, -propylat, -isopropylat, -butylat, -isobutylat, -sec-butylat und -tert-butylat. Besonders bevorzugt sind Natriumhydrid und Kalium-tert-butylat.

Die Reaktionstemperaturen können bei dem erfindungsgemäßen Verfahren in einem größeren Bereich variiert werden Im allgemeinen arbeitet man bei Temperaturen zwischen - 20 °C und + 100 °C, vorzugsweise bei Temperaturen zwischen - 5 °C und + 50 °C, insbesondere bei Temperaturen zwischen 0° C und 30° C.

Das erfindungsgemäße Verfahren wird im allgemeinen unter Normaldruck durchgeführt. Es ist jedoch auch möglich, unter erhöhtem oder vermindertem Druck -im allgemeinen zwischen 1 hPa und 2000 hPa, insbesondere zwischen 100 hPa und 1500 hPa zu arbeiten.

Zur Durchführung des erfindungsgemäßen Verfahrens setzt man auf 1 Mol 1-Heterocyclyl-2,4-difluor-5-nitro-benzol-Derivat der Formel (II) im allgemeinen zwischen 0,9 und 1,5 Mol, vorzugsweise zwischen 1,0 und 1,2 Mol, Hydroxyessigsäure-Derivat der Formel (III) bzw. dessen Alkalimetallsalz ein.

Die Alkalimetallsalze der Verbindungen der Formel (III) können entweder vor der erfindungsgemäßen Umsetzung in einem separaten Ansatz oder direkt im Reaktionsansatz erzeugt werden, wobei jeweils die oben angegebenenen Alkalimetall-Basen als Säurebindemittel verwendet werden. Zur Durchführung des erfindungsgemäßen Verfahrens können die Reaktionskomponenten in beliebiger Reihenfolge zusammen gegeben werden.

In einer bevorzugten Ausführungsform des erfindungsgemäßen Verfahrens wird das 1-Heterocyclyl-2,4-difluor-5-nitro-benzol-Derivat der Formel (II) in einem Verdünnungsmittel vorgelegt, das Hydroxyessigsäure-Derivat der Formel (III) bzw. dessen Alkalimetallsalz dazugegeben und dann gegebenenfalls ein Säureakzeptor langsam zudosiert. Das Reaktionsgemisch wird dann bis zum Ende der Umsetzung gerührt. Die Aufarbeitung kann nach üblichen Methoden durchgeführt werden. Beispielsweise wird nach dem Ende der Umsetzung im Wasserstrahlvakuum eingeengt und der Rückstand mit Wasser verrührt. Falls hierbei das Produkt der Formel (I) kristallin anfällt wird es durch Absaugen isoliert. Andernfalls wird mit einem mit Wasser praktisch nicht mischbarem organischen Lösungsmittel, wie z.B. Methylenchlorid, extrahiert, die organische Phase mit Wasser gewaschen, getrocknet, filtriert und eingeengt, wobei das Produkt der Formel (I) als Rückstand verbleibt.

Die nach dem erfindungsgemäßen Verfahren herzustellenden (5-Fluor-2-nitro-4-heterocyclyl-phenoxy)-essigsäure-Derivate können zur Herstellung von herbizid wirksamen 5-Fluor-6-heterocyclyl-3,4-dihydro-3-oxo-2H-1,4-benzoxazin-Derivaten der allgemeinen Formel (VII)

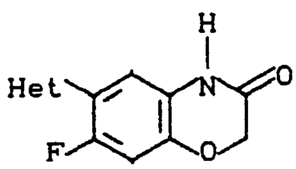

(VII)

in welcher
Het die oben angegebene Bedeutung hat,
verwendet werden.

Hierzu werden die Verbindungen der Formel (I) mit Reduktionsmitteln, wie z.B. Wasserstoff in Gegenwart eines Katalysators wie Raney-Nickel oder Eisen oder Zinn(II)-chlorid in Gegenwart einer Säure wie Salzsäure, vorzugsweise in Gegenwart von Verdünnungsmitteln, wie Ethanol und Wasser, bei Temperaturen zwischen 0 °C und 100 °C unter gleichzeitigem Ringschluß umgesetzt und nach üblichen Methoden aufgearbeitet.

Die Verwendbarkeit von Verbindungen der Formel (VII) als Herbizide ist bereits bekannt (vgl. EP-A 170 191, EP-A 255 601) bzw. Gegenstand neuerer Patentanmeldungen der Anmelderin (vgl. DE-P 38 32 348 vom 23.09.88 oder DE-P 38 36 742.4 vom 28.10.88).

Herstellungsbeispiele

Beispiel 1

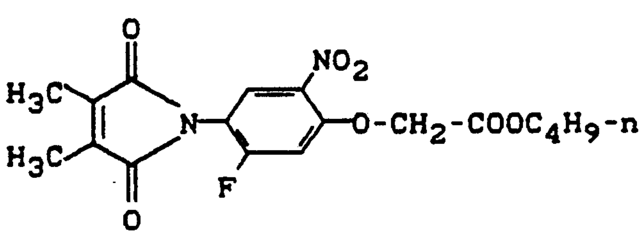

16 g (0,06 Mol) N-(2,4-Difluor-5-nitro-phenyl)-dimethylmaleinsäureimid werden in 250 ml absolutem

Tetrahydrofuran gelöst und 9 g (0,066 Mol) Glykolsäurebutylester hinzugefügt. Man kühlt auf 0 °C und gibt bei dieser Temperatur 2 g Natriumhydrid (80 %ig in Mineralöl) zu. Man rührt 4 Stunden bei 0 °C und anschließend über Nacht bei Raumtemperatur. Man engt ein und nimmt den Rückstand mit einem Gemisch aus Methylenchlorid und Wasser auf. Die Wasserphase wird abgetrennt und noch zweimal mit Methylenchlorid extrahiert Die vereingten organischen Phasen werden mit Wasser gewaschen, getrocknet und eingeengt.

Man erhält 20,1 g (85 % der Theorie) N-[2-Fluor-4-(butoxycarbonylmethoxy)-5-nitro-phenyl]-dimethylmaleinsäureimid vom Schmelzpunkt 98-100 °C.

$^1$H-NMR (CDCl): 0,93 ppm (t) $CH_3$; 1,4 ppm (m) $CH_2$;
1,65 ppm (m) $CH_2$; 2,1 ppm (s) $2CH_3$;
4,24 ppm (t) $CH_2$; 4,8 ppm (s) $CH_2$;
6,82 und 6,85 ppm (CH);
7,94 und 7,97 ppm (CH).

Beispiel 2

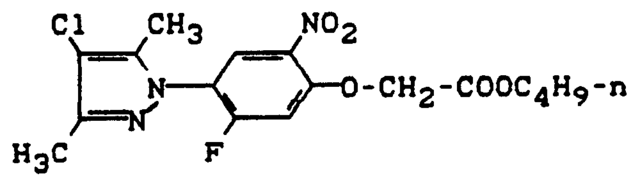

Eine Lösung aus 5,7 g (0,02 Mol) 1-(2,4-Difluor-5-nitrophenyl)-3,5-dimethyl-4-chlor-pyrazol, 100 ml Tetrahydrofuran und 2,9 g (0,022 Mol) Glykolsäurebutylester wird bei 0 °C mit 0,7 g (0,024 Mol) 80 %igem Natriumhydrid versetzt. Anschließend läßt man 4 Stunden bei 0 °C und 16 Stunden bei Raumtemperatur nachrühren. Zur Aufarbeitung wird die Reaktionsmischung im Vakuum eingeengt, der Rückstand mit Wasser verrührt, abgesaugt und getrocknet.

Man erhält 7,1 g (89 % der Theorie) an 1-(2-Fluor-4-butoxycarbonylmethoxy-5-nitro-phenyl )-3,5-dimethyl-4-chlor-pyrazol vom Schmelzpunkt 105 °C (langsame Zersetzung).

Ausgangsstoffe der Formel (II)

Beispiel (II-1)

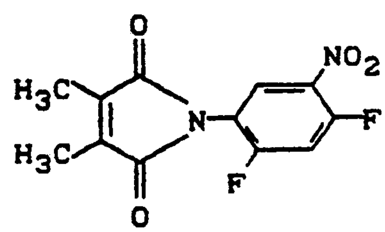

17,4 g (0,1 Mol) 2,4-Difluor-5-nitro-anilin und 12,6 g (0,1 Mol) Dimethylmaleinsäureanhydrid werden in 150 ml Essigsäure 16 Stunden unter Rückfluß gerührt. Nach dem Abkühlen wird mit 400 ml Wasser verdünnt und das ausgefallene Produkt abfiltriert, mit 1N-Salzsäure verrührt, erneut abfiltriert und getrocknet.

Man erhält 18,1 g N-(2,4-Difluor-3-nitro-phenyl)-dimethylmaleinsäureimid vom Schmelzpunkt 139,5-140,5 °C. Ausbeute: 64 % der Theorie.

Beispiel (II-2)

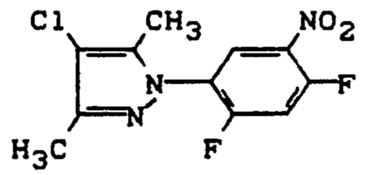

Zu einer Lösung aus 2,4 g (0,01 Mol) 1-(2,4-Difluor-phenyl)-3,5-dimethyl-4-chlor-pyrazol in 30 ml konzentrierter Schwefelsäure werden bei Raumtemperatur 1,0 ml (0,024 Mol) 98 %ige Salpetersäure getropft. Man läßt über Nacht bei Raumtemperatur nachrühren. Zur Aufarbeitung wird das Reaktionsgemisch in Wasser unter Kühlung eingetragen, eine halbe Stunde nachgerührt, abgesaugt und der Nutschenkuchen getrocknet.

Man erhält 2,7 g (94 % der Theorie) an 1-(2,4-Difluor-5-nitro-phenyl)-3,5-dimethyl-4-chlor-pyrazol vom Schmelzpunkt 134 °C.

Beispiel (II-3)

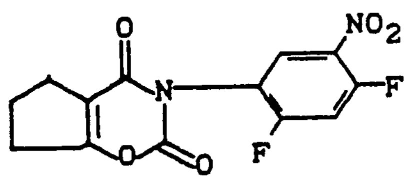

4,7 g (0,014 Mol; Gehalt 92,5 %) 3-(2,4-Difluor-phenyl)-2,3,4,5,6,7-hexahydrocyclopenta[e]-1,3-oxazin-2,4-dion werden in 40 ml Schwefelsäure gelöst. Man tropft bei 5 °C 1,5 g 65 %ige Salpetersäure, gelöst in 5 ml konzentrierter Schwefelsäure, zu und rührt eine Stunde bei 5-10 °C nach. Das Reaktionsgemisch wird auf Eis gegossen und mit Methylenchlorid extrahiert. Die vereinigten Extrakte werden mit Bicarbonatlösung und Wasser neutral gewaschen, getrocknet und einrotiert.

Man erhält 4,1 g 3-(2,4-Difluor-5-nitro-phenyl)-2,3,4,5,6,7-hexahydrocyclopenta[e]-1,3-oxazin-2,4-dion vom Schmelzpunkt 128 °C.

Ausgangsstoffe der Formel (IV)

Beispiel (IV-1)

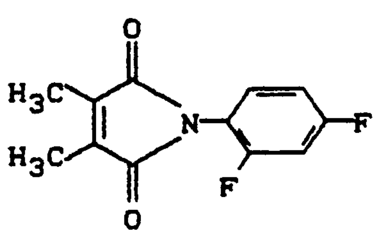

45,2 g (0,35 Mol) 2,4-Difluoranilin und 44,1 g (0,35 Mol) Dimethylmaleinsäureanhydrid werden in 500 ml Essigsäure 8 Stunden unter Rückfluß gerührt. Nach dem Abkühlen wird auf ca. 2 l Wasser gegossen und das ausgefallene Produkt abgesaugt. Man wäscht dreimal mit Wasser nach und trocknet.

Man erhält 62,5 g (75,3 der Theorie) N-(2,4-Difluor-phenyl)-dimethylmaleinsäureimid vom Schmelzpunkt 105-106 °C in Form hellbeiger Kristalle.

Beispiel (IV-2)

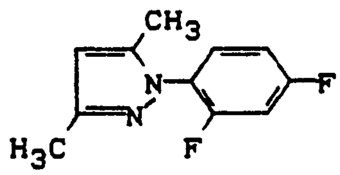

Eine Lösung aus 7,2 g (0,05 Mol) 2,4-Difluor-phenylhydrazin, 150 ml Ethanol und 5,0 g (0,05 Mol) 2,4-Pentandion wird 24 Stunden unter Rückfluß erhitzt. Zur Aufarbeitung wird im Vakuum das Lösungsmittel entfernt, der Rückstand in Methylenchlorid aufgenommen, 2 mal mit Wasser gewaschen, über Natriumsulfat getrocknet und das Lösungsmittel im Vakuum abdestilliert. Anschließend wird der Rückstand auf Ton getrocknet.

Man erhält 7,5 g (72 % der Theorie) 1-(2,4-Difluor-phenyl)-3,5-dimethyl-pyrazol vom Schmelzpunkt 57 °C.

Beispiel (IV-3)

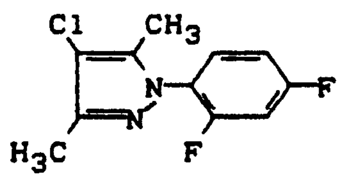

4,2 g (0,02 Mol) 1-(2,4-Difluor-phenyl)-3,5-dimethylpyrazol in 100 ml Methylenchlorid werden mit 1,6 ml (0,02 Mol) Sulfurylchlorid versetzt. Man läßt 16 Stunden bei Raumtemperatur nachrühren. Anschließend wird die Reaktionslösung über Natriumsulfat filtriert, das Filtrat im Vakuum eingeengt, der Rückstand in Ether aufgenommen, wiederum filtriert und die Etherphase im Vakuum eingeengt.

Man erhält 3,5 g (72 % der Theorie) an 1-(2,4-Difluorphenyl)-3,5-dimethyl-4-chlor-pyrazol vom Schmelzpunkt 89 °C.

Beispiel IV-4

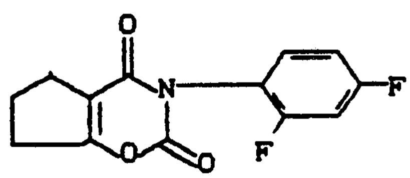

3,1 g (0,02 Mol) 2,4-Difluor-phenylisocyanat werden in 15 ml o-Dichlorbenzol gelöst und 3,4 g (0,02 Mol) Acetonid der 2-Hydroxy-cyclopenten-1-carbonsäure (d.h. 3-(2,4-Difluor-phenyl)-2,3,4,5,6,7-hexahydrocyclopenta[e]-1,3-dioxin-4-on) in 10 ml o-Dichlorbenzol bei 150 °C unter Rühren zugetropft. Es wird 4 Stunden bei dieser Temperatur gehalten und dann das Lösungsmittel unter vermindertem Druck abdestilliert.

Man erhält 4,7 g eines bräunlichen Öls, welches direkt in die nächste Stufe (Nitrierung) eingesetzt werden kann.

2,4-Difluor-5-nitro-anilin

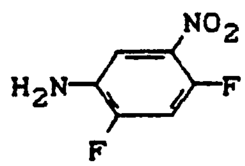

150 g (1,15 Mol) 2,4-Difluor-anilin werden unter Kühlung (-5 bis 0 °C) in 680 ml konzentrierter Schwefelsäure gelöst. Bei -5 °C Innentemperatur tropft man anschließend eine Lösung von 53 ml (1,28 Mol) konzentrierter Salpetersäure in 280 ml konzentrierter Schwefelsäure ca. 30 Minuten zu, läßt dann die Innentemperatur in ca. 2 Stunden auf 0 °C steigen und rührt 3,5 Stunden bei dieser Temperatur nach. Zur Aufarbeitung wird die Reaktionsmischung in ca. 1,5 kg Eiswasser eingerührt und der Niederschlag abgesaugt.

Man erhält 170 g 2,4-Difluor-5-nitro-anilin vom Schmelzpunkt 94-96 °C. Ausbeute: 84,5 % der Theorie.

Wirkstoffe der Formel (VII)

Beispiel (VII-1)

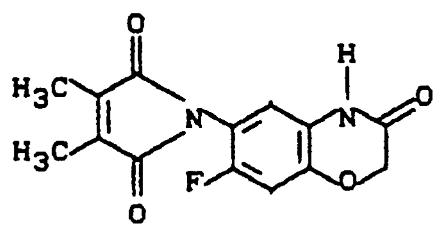

3,94 g (0,1 Mol) N-[2-Fluor-4-(butoxycarbonylmethoxy)-5-nitro-phenyl]-dimethylmaleinimid werden in 100 ml Ethanol gelöst und dann bei 20-30 °C mit einer Lösung von 7,5 g Zinn(II)-chlorid (Dihydrat) in 60 ml konzentrierter Salzsäure tropfenweise versetzt. Es wird 2 Stunden bei 50-60 °C gerührt, abgekühlt und das Lösungsmittel abdestilliert. Man versetzt mit Wasser und filtriert ab. Der Rückstand wird mit Wasser und kaltem Ethanol gewaschen und getrocknet. Nach Umkristallisieren aus Ethanol erhält man 2,3 g (79,3 % der Theorie) 7-Fluor-6-dimethylmaleinimido-3,4-dihydro-3-oxo-2H-1,4-benzoxazin vom Schmelzpunkt 226-228 °C.

Beispiel (VII-2)

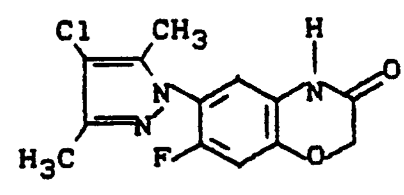

Zu einer Lösung aus 6 ml Ethanol 2,3 ml Wasser, 0,15 ml konzentrierter Salzsäure und 1,9 g (0,0335 Mol) Eisenpulver, die zuvor 30 Minuten unter Rückfluß erhitzt wurden, werden bei 65-70 °C innerhalb von 2 Stunden 3,0 g (0,0075 Mol) 1-(2-Fluor-4-butoxycarbonylmethoxy-5-nitro-phenyl)-3,5-dimethyl-4-chlor-pyrazol gelöst in 30 ml Ethanol getropft und die Mischung wird 2 Stunden unter Rückfluß gerührt. Anschließend werden bei 70 °C 0,3 g Soda zugesetzt und 30 Minuten unter Rückfluß gerührt. Zur Aufarbeitung wird heiß abgesaugt, dreimal mit je 5 ml Ethanol nachgewaschen und das Filtrat im Vakuum eingeengt. Der Rückstand wird mit Petrolether verrührt und abgesaugt.

Man erhält 1,9 g (86 % der Theorie) 7-Fluor-6-(3,5-dimethyl-4-chlor-pyrazol)-3,4-dihydro-3-oxo-2H-1,4-benzoxazin vom Schmelzpunkt 236 °C.

**Ansprüche**

1. Verfahren zur Herstellung von (5-Fluor-2-nitro-4-heterocyclylphenoxy)-essigsäure-Derivaten der allgemeinen Formel (I)

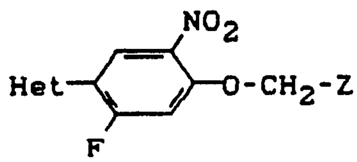

(I)

in welcher
Z für Cyano, Carboxy oder Alkoxycarbonyl steht und
Het für eine der nachstehenden heterocyclischen Gruppierungen steht

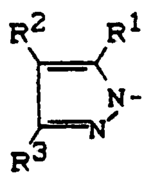 , 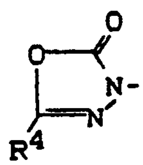 , 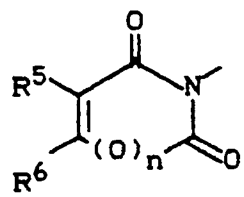

worin
$R^1$ für Wasserstoff, Halogen oder gegebenenfalls durch Halogen substituiertes Alkyl steht,
$R^2$ für Wasserstoff, Cyano, Nitro, Halogen oder gegebenenfalls durch Halogen substituiertes Alkyl steht,
$R^3$ für Wasserstoff oder gegebenenfalls durch Halogen substituiertes Alkyl steht oder zusammen mit $R^2$ für Alkandiyl steht,
$R^4$ für Wasserstoff oder für jeweils gegebenenfalls verzweigtes und gegebenenfalls durch Halogen substituiertes Alkyl, Cycloalkyl, Alkenyl oder Alkinyl steht,
$R^5$ für Wasserstoff, Halogen oder gegebenenfalls durch Halogen substituiertes Alkyl steht,
$R^6$ für Wasserstoff, Halogen oder gegebenenfalls durch Halogen substituiertes Alkyl steht, oder zusammen mit $R^5$ für Alkandiyl steht und
n für die Zahlen 0 oder 1 steht,
dadurch gekennzeichnet, daß man 1-Heterocyclyl-2,4-difluor-5-nitrobenzol-Derivate der allgemeinen Formel (II)

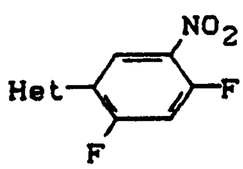

(II)

in welcher
Het die oben angegebene Bedeutung hat,
mit Hydroxyessigsäure-Derivaten der allgemeinen Formel (III)
$HO-CH_2-Z$ (III)
in welcher
Z die oben angegebene Bedeutung hat,
oder mit Alkalimetallsalzen von Verbindungen der Formel (III)
in Gegenwart eines Verdünnungsmittels umsetzt.

2. Verfahren gemäß Anspruch 1, dadurch gekennzeichnet, daß in Formel (I)
Z für Cyano, Carboxy oder $C_1$-$C_4$-Alkoxycarbonyl steht und

Het für eine der nachstehenden heterocyclischen Gruppierungen steht

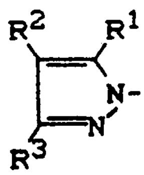 , 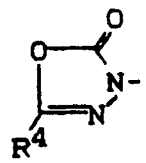 , 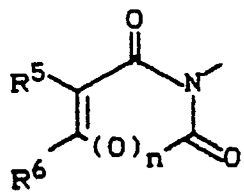

worin

$R^1$ für Wasserstoff, Fluor, Chlor, Brom oder für gegebenenfalls durch Fluor und/oder Chlor substituiertes $C_1$-$C_4$-Alkyl steht,

$R^2$ für Wasserstoff, Cyano, Nitro, Fluor, Chlor, Brom, Iod oder für gegebenenfalls durch Fluor und/oder Chlor substituiertes $C_1$-$C_4$-Alkyl steht,

$R^3$ für Wasserstoff oder für gegebenenfalls durch Fluor und/oder Chlor substituiertes $C_1$-$C_4$-Alkyl steht, oder zusammen mit $R^2$ für $C_3$- oder $C_4$-Alkandiyl steht,

$R^4$ für Wasserstoff oder für jeweils gegebenenfalls verzweigtes und gegebenenfalls durch Fluor, Chlor und/oder Brom substituiertes $C_1$-$C_4$-Alkyl, $C_3$-$C_6$-Cycloalkyl, $C_2$-$C_4$-Alkenyl oder $C_2$-$C_4$-Alkinyl steht,

$R^5$ für Wasserstoff, Fluor, Chlor, Brom oder für gegebenenfalls durch Fluor und/oder Chlor substituiertes $C_1$-$C_4$-Alkyl steht,

$R^6$ für Wasserstoff, Fluor, Chlor, Brom oder für gegebenenfalls durch Fluor und/oder Chlor substituiertes $C_1$-$C_4$-Alkyl steht, oder zusammen mit $R^5$ für $C_3$- oder $C_4$-Alkandiyl steht und

n für die Zahlen 0 oder 1 steht.

3. Verfahren gemäß Anspruch 1, dadurch gekennzeichnet, daß in Formel (I)

Z für Methoxycarbonyl, Ethoxycarbonyl, n- oder iso-Propoxycarbonyl, n-, iso-, sec- oder tert. Butoxycarbonyl steht und

Het für eine der nachstehenden heterocyclischen Gruppierungen steht

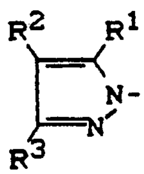 , 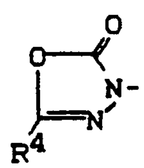 , 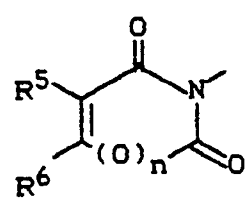

worin

$R^1$ für Wasserstoff, Chlor, Methyl, Ethyl oder Trifluormethyl steht,

$R^2$ für Wasserstoff, Chlor, Brom, Methyl oder Trifluormethyl steht,

$R^3$ für Wasserstoff, Methyl, Ethyl oder Trifluormethyl steht, oder zusammen mit $R^2$ für Trimethylen oder Tetramethylen steht,

$R^4$ für Wasserstoff oder für jeweils gegebenenfalls verzweigtes und gegebenenfalls durch Fluor und/oder Chlor substituiertes $C_1$-$C_4$-Alkyl oder $C_3$-$C_5$-Cycloalkyl steht,

$R^5$ für Wasserstoff, Chlor oder Methyl steht,

$R^6$ für Wasserstoff, Chlor oder Methyl steht oder zusammen mit $R^5$ für Trimethylen oder Tetramethylen steht und

n für die Zahlen 0 oder 1 steht.

4. Verfahren gemäß Anspruch 1, dadurch gekennzeichnet, daß man die Umsetzung in Gegenwart von Säurebindemitteln durchführt.

5. Verfahren gemäß Anspruch 1, dadurch gekenneichnet, daß die Umsetzung bei Temperaturen zwischen -20 °C und +100 °C durchgeführt wird.

6. Verfahren gemäß Anspruch 1, dadurch gekennzeichnet, daß man auf 1 Mol 1-Heterocyclyl-2,4-difluor-5-nitro-benzol-Derivat der Formel (II) zwischen 0,9 und 1,5 Mol Hydroxyessigsäure-Derivat der Formel (III) bzw. dessen Alkalimetallsalz einsetzt.

7. 1-Heterocyclyl-2,4-difluor-5-nitrobenzol-Derivate der Formel (II)

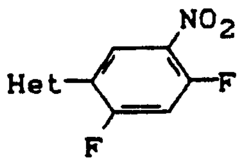

(II)

in welcher
Het für eine der nachstehenden heterocyclischen Gruppierungen steht

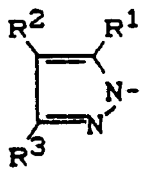 , 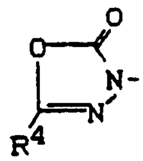 , 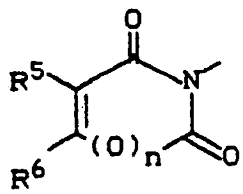

worin
$R^1$ für Wasserstoff, Halogen oder gegebenenfalls durch Halogen substituiertes Alkyl steht,
$R^2$ für Wasserstoff, Cyano, Nitro, Halogen oder gegebenenfalls durch Halogen substituiertes Alkyl steht,
$R^3$ für Wasserstoff oder gegebenenfalls durch Halogen substituiertes Alkyl steht oder zusammen mit $R^2$ für Alkandiyl steht,
$R^4$ für Wasserstoff oder für jeweils gegebenenfalls verzweigtes und gegebenenfalls durch Halogen substituiertes Alkyl, Cycloalkyl, Alkenyl oder Alkinyl steht,
$R^5$ für Wasserstoff, Halogen oder gegebenenfalls durch Halogen substituiertes Alkyl steht,
$R^6$ für Wasserstoff, Halogen oder gegebenenfalls durch Halogen substituiertes Alkyl steht, oder zusammen mit $R^5$ für Alkandiyl steht und
n für die Zahlen 0 oder 1 steht.